# EUROPÄISCHE PATENTSCHRIFT

(11) **EP 1 056 460 B1**
(45) Veröffentlichungstag und Bekanntmachung des Hinweises auf die Patenterteilung: **22.10.2003**
(21) Anmeldenummer: 99910118.1
(22) Anmeldetag: 10.02.1999
(51) Int. Cl.: A61K 31/565, A61K 31/57, A61P 25/00, A61P 5/24, A61P 15/12

(54) **PHARMAZEUTISCHE PRÄPARATE ZUR GEZIELTEN SUBSTITUTION DES ESTROGENMANGELS IM ZENTRALNERVENSYSTEM**
PHARMACEUTICAL PREPARATIONS FOR SELECTIVELY SUPPLEMENTING OESTROGEN DEFICIENCY IN THE CENTRAL NERVOUS SYSTEM
PREPARATIONS PHARMACEUTIQUES POUR PALLIER DE FA ON CIBLEE LA CARENCE EN OESTROGENES DU SYSTEME NERVEUX CENTRAL

(30) Priorität: 20.02.1998 DE 19807264; 15.05.1998 DE 19821831
(43) Veröffentlichungstag der Anmeldung: 06.12.2000
(73) Patentinhaber: Schering AG, 13353 Berlin (DE)
(72) Erfinder: PATCHEV, Vladimir, Dr., D-07749 Jena (DE); OETTEL, Michael, Prof.Dr., D-07743 Jena (DE); THIEME, Ina, D-07612 Graitschen (DE); SCHWARZ, Sigfrid, Prof.Dr., D-07743 Jena (DE); RÖMER, Wolfgang, Prof.Dr., D-07749 Jena (DE)
(74) Vertreter: Cramer, Eva-Maria
(86) Internationale Anmeldenummer: DE9900353
(87) Internationale Veröffentlichungsnummer: WO99042108

(56) Entgegenhaltungen:
- WO-A-95/12402
- WO-A-97/03661
- DE-A- 4 239 946
- DE-A- 4 429 397
- DE-A- 19 524 937
- DE-C- 4 338 314
- US-A- 4 791 099

## Beschreibung

Die Erfindung betrifft die Verwendung von ausgewählten Steroiden zur Herstellung pharmazeutischer Präparate zur gezielten Substitution des Estrogenmangels im ZNS ohne Beeinflussung anderer Organe oder Systeme.

Diese Steroide zeichnen sich dadurch aus, daß sie im Gegensatz zu systemisch wirksamen natürlichen und synthetischen Estrogenen, inklusive 17a-Estradiol, selektive neurotrope estrogen-ähnliche Transkriptionswirkung besitzen.

Diese Steroide sind Verbindungen der allgemeinen Formel I 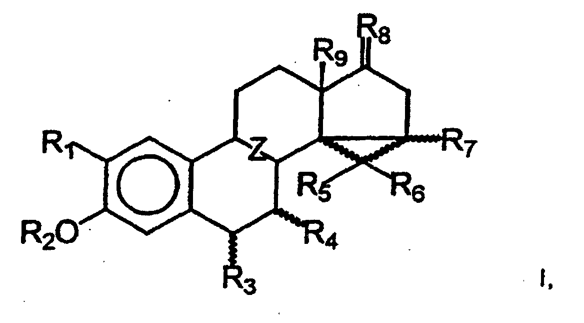 in der R₁ ein Wasserstoffatom, eine Hydroxylgruppe oder eine Alkyloxygruppe von 1 bis 5 C-Atomen darstellt, R₂ ein Wasserstoffatom, eine Alkylgruppe von 1 bis 5 C-Atomen, eine Acylgruppe von 1 bis 5 C-Atomen, eine Gruppierung der allgemeinen Formel SO₂NR₁₀R₁₁, wobei R₁₀ und R₁₁ unabhängig voneinander jeweils ein Wasserstoffatom, eine Alkylgruppe von 1 bis 5 C-Atomen oder zusammen mit dem Stickstoff eine Pyrrolidino-, Piperidino- oder Morpholinogruppe bedeuten, R₃ ein Wasserstoffatom oder eine Hydroxylgruppe darstellt, R₄ ein Wasserstoffatom, eine Hydroxylgruppe oder eine Alkylgruppe bis 5 C-Atomen bedeutet, R₅ und R₆ unabhängig voneinander jeweils ein Wasserstoffatom oder ein Halogenatom bedeuten, R₇ für ein Wasserstoffatom oder eine Methylgruppe steht, R₈ ein Wasserstoffatom und eine Hydroxylgruppe, ein Oxogruppe oder eine Gruppierung der allgemeinen Formel CR₁₂R₁₃ bedeutet, in der R₁₂ und R₁₃ unabhängig voneinander jeweils ein Wasserstoffatom oder ein Halogenatom darstellen, R₉ eine Methyloder Ethylgruppe bedeutet, Z für eine C,C-Doppelbindung oder einen substituierten oder unsubstituierten Cyclopropanring steht und die Gruppierung >CR₅R₆ entweder α-oder β-ständig angeordnet ist, wobei R₇ β-ständig ist, wenn >CR₅R₆ α-ständig ist und umgekehrt.

Eine abrupte oder allmähliche Abnahme der Estrogen-Konzentrationen im Organismus kann sowohl bei Frauen als auch bei Männern unter physiologischen (zunehmendes Alter, Menopause) und pathologischen Bedingungen (Gonadektomie, Einsatz von GnRH-Analoga als supplementäre Krebstherapie) auftreten.
Zu den bekanntesten klinischen Symptomen des Estrogen-Ausfalls gehören Störungen der Thermoregulation in der Form von Hitzewallungen, Osteoporose und erhöhte Prädisposition zu Herz- und Kreislauf-Erkrankungen (Netter A, The menopause. In: Thibault C, Levasseur MC, Hunter RHF (eds), Reproduction in Mammals and Man, Ellipses, Paris, 627-642, 1993).
Neueste klinische Studien (van den Beld AW et al.,The role of estrogens in physical and psychosocial well-being in elderly men, The Aging Male 1 (Suppl. 1), 54, 1998) haben eindeutige Beweise für eine Senkung der Serum-Estrogenspiegel mit zunehmendem Alter beim Mann erbracht. Dadurch wird das Vorhandensein und die pathophysiologische Relevanz eines "Estrogen-Mangelsyndroms" beim alternden Mann unterstrichen.

Das Gehirn stellt ein sehr wichtiges Zielorgan der Estrogenwirkung dar. Estrogene haben einen entscheidenden physiologischen Einfluß auf viele neurobiologische Prozesse. Ihre Effekte lassen sich im allgemeinen in zwei großen Gruppen -organisierende und aktivierende - klassifizieren ( McEwen BS et al., Steroid hormones as mediators of neural plasticity, J Steroid Biochem Mol Biol 39: 223-232, 1991).

Die Ersteren betreffen hauptsächlich die geschlechtsspezifische Organisation neuraler Substrate während der frühen Ontogenese.
Die zweite Gruppe umfaßt spezifische Veränderungen in der Funktion neuraler Regelkreise unter dem Einfluß von Estrogenkonzentrationen, die aus der physiologischen Sekretion der Gonaden nach der Geschlechtsreife resultieren. Die aktivierenden Effekte von Estrogenen im ZNS kommen u.a. bei den folgenden physiologischen Prozessen zum Ausdruck:
geschlechtsspezifische Regulation der Gonadotropin-Sekretion (Fink G, Gonadotropin secretion and ist control. In: Knobil E, Neil JD (eds), The Physiology of Reproduction, Raven Press, New York, 1349-1376, 1988),
Steuerung des Sexualverhaltens (Baum MJ et al., Hormonal basis of proceptivity and receptivity in female primates, Arch Sex Behav 6: 173-192, 1977),
Regulation der neuroendokrinen Reagibilität auf Streß (Viau V, Meaney MJ, Variations in the hypothalamic-pituitary-adrenal response to stress during the estrous cycle in the rat, Endocrinology 129: 2503-2511, 1991),
Lernen und Retention von Verhaltensmustern mit adaptiver Relevanz (O'Neal MF et al., Estrogen affects performance of ovariectomized rats in a two-choice water-escape working memory task, Psychoneuroendocrinology 21: 51-65, 1996).
Aufrechterhaltung der Reaktionsbereitschaft von neurochemischen Mechanismen, die für die Gewährleistung der Vigilanz und adäquaten Informationsverarbeitung unenthehrlich sind (Fink G et al., Estrogen control of central neurotransmission: effects on mood, mental state and memory, Cell Mol Neurobiol 16: 325-344, 1996).
dynamische Veränderungen der Dichte interneuronaler Kontakte in Hirnstrukturen mit entscheidender Rolle für die kognitive Leistung und den emotionalen Status ( Wooley CS, McEwen BS, Estradiol mediates fluctuations in hippocampal synapse density during the estrous cycle in the adult rat. J Neurosci 12: 2549-2554, 1992).
Das enorme neurotrope Potential von Estrogenen findet einen Ausdruck in ihrer Fähigkeit,
die Expression von einer Reihe von ZNS-spezifischen Genen zu induzieren, deren Produkte für das Überleben von Nervenzellen von kritischer Bedeutung sind (Miranda RC, Sohrabji F, Toran-Allerand CD, Presumptive estrogen target neurons express mRNA for both the neurotrophins and neurotrophin receptors: a basis for potential development interactions of estrogen with the neurotrophins, Mol Cell Neurosci 4: 510-525, 1993),
die Vielfalt und Qualität der Signalübertragung im ZNS (Luine VN Estradiol increases choline acetyltransferase activity in specific basal forebrain nuclei and projection areas of female rats, Exp Neurol 89: 489-490, 1985); (Weiland N, Glutamic acid decarboxylase messenger ribonucteic acid is regulated by estradiol and progesterone in the hippocampus, Endocrinology 131: 2697-2702, 1992); (Bossé R, Di Paolo T, The modulation of brain dopamine and GABA_{A} receptors by estradiol: a clue for CNS changes occurring at menopause, Cell Mol Neurobiol 16: 199-212, 1996), zu gewährleisten
und die Resistenz von Nervenzellen gegenüber pathologischen Einwirkungen zu erhöhen (Goodman Y et al., Estrogens attenuate and corticosterone exacerbates excitotoxicity, oxidative injury, and amyloid b-peptide toxicity in hippocampal neurons, J Neurochem 66: 1836-1844, 1996).

Klinische Befunde implizierten den Estrogenmangel als kausalen Faktor in der Pathogenese des Morbus Alzheimer und deuten auf die Möglichkeit einer Estrogen-Substitution, die klinische Manifestation bzw. Progredienz dieser Erkrankung aufzuhalten (Henderson VW et al., Estrogen replacement therapy in older women: comparisons between Alzheimer's disease cases and controls, Arch Neurol 51: 896-900, 1994); (Paganini-Hill A, Henderson VW, Estrogen deficiency and risk of Alzheimer's disease, Am J Epidemiol, 140: 256-261, 1994).
Eine Reihe von Neuropeptiden, deren Gentranskription durch physiologische Estrogenmengen beeinflusst wird (z.B. Oxytozin und Arginin-Vasopressin) spielen eine wichtige Rolle bei der Steuerung emotionaler Verhaltenskomponenten (Adan RA, Burbach JP, Regulation of vasopressin and oxytocin gene expression by estrogen and thyroid hormone, Progr Brain Res 92: 127-136, 1992).

Berichte in der Fachliteratur weisen darauf hin, daß Estrogenmangel mit einer deutlichen Abschwächung der Fähigkeit des Organismus, reaktive Sauerstoffspezies und freie Radikale zu eliminieren, einhergeht (Niki E, Nakano M, Estrogens as antioxidants. Methods Enzymol 186: 330-333, 1990) ; (Lacort M et al., Protective effects of estrogens and catecholestrogens against peroxidative membrane damage in vitro, Lipids 30: 141-146, 1995). Der Überschuß an freien Radikalen wird in Mechanismen der zellulären Schädigung in mehreren Organen und Systemen impliziert und mit der Pathogenese von neurodegenerativen Erkrankungen im Zusammenhang gebracht (Smith CD et al., Excess brain protein oxidation and enzyme dysfunction in normal aging and in Alzheimer's disease. Proc Natl Acad Sci USA 88: 10540-10543, 1991); (Hastings TG, Zigmond MJ, Neurodegenerative disease and oxidative stress: insights from an animal model of Parkinsonism. In: Fiskum G (ed), Neurodegenerative Diseases, Plenum Press, New York, 37-46, 1996). Daher wird der Estrogen-Substitution auch eine Rolle im Sinne der Aufrechterhaltung und Erhöhung der endogenen antioxidativen Kapazität beigemessen (Behl C et al., 17b-Estradiol protects neurons from oxidative stress-induced cell death in vitro, Biochem Biophys Res Commun 216: 473-482. 1995).

Zum gegenwärtigen Zeitpunkt erfolgt die Estrogen-Substitution mit natürlichen und synthetischen Estrogenen, deren Wirkung in allen Estrogerirezeptor-enthaltenden Organen und Systemen auftritt, d.h. praktisch im gesamten Körper. Da jedoch diese Estrogene bereits in geringen pharmakologischen Dosen eine starke Zellproliferation in Geweben des weiblichen Genitaltraktes (Endometrium) und dem Brustdrüsenepithel verursachen, die schließlich in einer karzinogener Entdifferenzierung ausarten, ist ihre Anwendung für die Therapie von Symptomen eines Estrogen-Mangels im ZNS durch mehrere Gegenindikationen begrenzt (Bernstein BA, Ross RK, Henderson BE, Relationship of hormone use to cancer risk. J Natl Cancer Inst Monograph 12: 137-147, 1992).

Die proliferativen Effekte von Estrogenen gelten als unmittelbare Risikofaktoren für die Entstehung einer benignen Prostata-Hyperplasie und/oder Gynäkomastie beim Mann (Knabbe C, Endokrine Therapie von Prostataerkrankungen. In: Allolio B Schulte HM (eds), Praktische Endokrinologie, Urban & Schwarzenberg, München, 645-651, 1996).
Aus diesem Grund wurde die Estrogen-Substitution beim Mann, trotz erwiesenen Indikationen, nie ernsthaft in Erwägung gezogen.

Die Verwendung von natürlichen und synthetischen Estrogenen mit systemischer Wirkung - d.h. in allen Organen und Systemen des Körpers - zur Behandlung von neurodegenerativen Erkrankungen wird durch die folgenden Patente beansprucht:
US 4,897,389, US 5,554,601 und WO 95/12402, WO 97/03661, DE 43 38 314 C1.
- US 4,897,389 schützt die Anwendung von Estradiol, Estron und Estriol, allein oder in Kombination mit Gonadotropinen, Androgenen, anabolen Androgenen oder humanem Wachstumshormon, zur Behandlung seniler Demenz, Morbus Parkinsoni, zerebraler Atrophie, Morbus Alzheimeri, zerebellarer Atrophie, senilem oder essentiellem Tremor.
- US 5,554,601 und WO 95/12402 schützt die Anwendung von estrogenen Substanzen, darunter auch solche, die eine geringfügige "sexuelle Aktivität" aufweisen, zur Protektion von Nervenzellen vor progredienter Schädigung und dem Zelltod, und zur Behandlung neurodegenerativer Erkrankungen. Als Beispiel für eine Substanz mit geringfügiger "sexueller Aktivität" und neuroprotektiver Wirkung wird 17a-Estradiol angeführt.
- WO 97/03661 schützt die Anwendung von nicht-estrogenen Substanzen, die in ihrer Struktur mindestens zwei Ringstrukturen aufweisen, wobei mindestens eine davon ein terminaler phenolischer Ring ist, und deren Molekülgewicht weniger als 1000 Dalton be trägt, zur Gewährleistung von Neuroprotektion.
- DE 43 38 314 C1 beschreibt Steroide mit phenolischer A-Ring-Struktur, deren radikalfangenden und antioxidativen Eigenschaften unabhängig vom Maß der estrogen-ähnlichen Wirksamkeit sind. Die se Verbindungen können zur Prophylaxe und Therapie radikalvermittelter Zellschädigungen eingesetzt werden.
In all diesen Patentschriften soll die therapeutische und neuroprotektive Effizienz der enthaltenen Substanzen auf einen oder mehrere der folgenden Endeffekten basieren:
- Stimulation der Biosynthese von natürlichen neuronalen Wachstumsfaktoren;
- Stimulation der Aktivität von Acetylcholin-synthetisierenden Enzymen bzw. der Aufnahme (Uptake) von Substraten der Acetylcho-lin-Synthese;
- direkte Zytoprotektion durch Erhöhung der Resistenz von Nervenzel len gegenüber dem Entzug von Nährsubtraten bzw. Wachstumsfak toren;
- Verminderung der Empfindlichkeit von Nervenzellen gegenüber freien Radikalen und reaktiven Sauerstoff-Spezies, die infolge einer traumatischen bzw. neurotoxischen Einwirkung freigesetzt werden.

Jedoch werden in keiner der aufgeführten Patentschriften Steroide mit selektiven estrogen-ähnlichen neurotropen Transkriptionseffekten dargestellt; d.h. solche, die bei einer Dosierung in vivo, die im reproduktiven System keine signifikante biologische Wirkung zeigen, die Transkription estrogen-abhängiger Gene im ZNS in einem estrogen-ähnlichen Modus beeinflussen.
Insbesondere gilt es zu unterstreichen, daß die in den Patentschriften US 5,554,601 und WO 95/12402 beschriebene Wirkung von 17a-Estradiol - einer Substanz, die eine reduzierte Estrogenität im Genitaltrakt aufweist (Clark JH et al., Effects of estradiol 17a on nuclear occupancy of the estrogen receptor, stimulation of nuclear type II sites, and uterine growth, J Steroid Biochem 16: 323-328, 1982) - sich nur auf die Protektion von kultivierten Nervenzellen vor dem durch den Entzug von Nährmittel induzierten Zelltod bezieht, wobei die relative Potenz von 17a-Estradiol mit derjenigen von 17b-Estradiol nicht evaluiert wurde. Daraus kann man schlußfolgern, daß aus den bisher veröffentlichten Untersuchungen und Patentschriften jegliche Hinweise für eine selektive neurotrope Wirkung von 17a-Estradiol bzw. seinen Derivaten fehlen, während 17b-Estradiol bekanntlich keine ZNS-Selektivität aufweist und damit als ein Estrogen mit systemischer Wirkung einzustufen ist. Die Patentschriften WO 97/03661 und DE 43 38 314 C1 interpretieren die zytoprotektive Wirkung von Estrogenen für eine Konsequenz der radikalfangenden Eigenschaften ihres terminalen phenolischen A-Rings. Eine dissoziierte neurotrope Wirkung von 17a-Estradiol, die auf einer Beeinflussung der Transkription von estrogen-sensitiven Genen basiert, wurde weder innerhalb der Patentschrift untersucht, noch wurde in der Literatur darüber berichtet.

Der Erfindung liegt die Aufgabe zugrunde, pharmazeutische Präparate zur gezielten Substitution des Estrogenmangels im Zentralnervensystem (ZNS) ohne Beeinflussung anderer Organe oder Systeme zu finden.

Die Aufgabe wird erfindungsgemäß dadurch gelöst, daß ausgewählte Steroide zur Herstellung pharmazeutischer Präparate verwendet werden, die die Substitution des Estrogenmangels im ZNS ohne Beeinflussung anderer Organe oder Systeme gewährleisten.

Die Steroide zeichnen sich dadurch aus, daß sie im Gegensatz zu systemisch wirksamen natürlichen und synthetischen Estrogenen, inklusive 17a-Estradiol, selektive neurotrope estrogen-ähnliche Transkriptionswirkung besitzen.

Es wurde überraschend festgestellt, daß die ausgewählten Steroide in ihrer erfindungsgemäßen Verwendung
- eine selektive Beeinflussung der Transkription estrogen-abhängiger Gene im ZNS und Veränderungen entsprechender physiologischer Parameter verursachen;
- ZNS-spezifische Transkriptionseffekte in solchen Dosen aufweisen, die in Geweben des Reproduktionssystems keine bioloische Effekte haben;
- ZNS-spezifischen Transkriptionseffeke bei solchen Dosierungen aufweisen, in denen weder 17b-Estradiol, noch 17a-Estradiol, eine Wirksamkeit zeigen;
- und die Transkription estrogen-abhängiger Gene im ZNS nicht über die Wirkung von sekundär gebildetem 17b-Estradiol beeinflussen.

Diese Steroide sind Verbindungen der allgemeinen Formel I 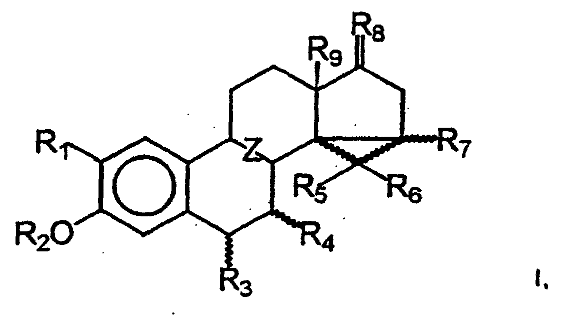 in der R₁ ein Wasserstoffatom, eine Hydroxylgruppe oder eine Alkyloxygruppe von 1 bis 5 C-Atomen darstellt, R₂ ein Wasserstoffatom, eine Alkylgruppe von 1 bis 5 C-Atomen, eine Acylgruppe von 1 bis 5 C-Atomen, eine Gruppierung der allgemeinen Formel SO₂NR₁₀R₁₁, wobei R₁₀ und R₁₁ unabhängig voneinander jeweils ein Wasserstoffatom, eine Alkylgruppe von 1 bis 5 C-Atomen oder zusammen mit dem Stickstoff eine Pyrrolidino-, Piperidino- oder Morpholinogruppe bedeuten, R₃ ein Wasserstoffatom oder eine Hydroxylgruppe darstellt, R₄ ein Wasserstoffatom; eine Hydroxylgruppe oder eine Alkylgruppe bis 5 C-Atomen bedeutet, R₅ und R₆ unabhängig voneinander jeweils ein Wasserstoffatom oder ein Halogenatom bedeuten, R₇ für ein Wasserstoffatom oder eine Methylgruppe steht, R₈ ein Wasserstoffatom und eine Hydroxylgruppe, ein Oxogruppe oder eine Gruppierung der allgemeinen Formel CR₁₂R₁₃ bedeutet, in der R₁₂ und R₁₃ unabhängig voneinander jeweils ein Wasserstoffatom oder ein Halogenatom darstellen, R₉ eine Methyloder Ethylgruppe bedeutet, Z für eine C,C-Doppelbindung oder einen substituierten oder unsubstituierten Cyclopropanring steht und die Gruppierung >CR₅R₆ entweder α-oder β-ständig angeordnet ist, wobei R₇ β-ständig ist, wenn >CR₅R₆ α-ständig ist und umgekehrt.

Bevorzugte Verbindungen sind
15βH,3'H-Cycloprop[14,15]-estra-1,3,5(10),8-tetraen-3,17α-diol,
15βH,3'H-Cycloprop[14,15]-18a-homo-estra-1,3,5(10),8-tetraen-3,17α-ol,
17α-Hydroxy-15βH,3'H-cycloprop[14,15]-estra-1,3,5(10),8-tetraen -3-ylpentanoat,
17-Methylen-15βH,3'H-cycloprop[14,15]-estra-1,3,5(10),8-tetraen-3-ol,
15βH,3'H-3',3'-difluoro-cycloprop[14,15]-estra-1,3,5(10),8-tetraen-3,17α-diol,
17-Methylen-15βH,3'H-cycloprop[14,15]-estra-1,3,5(10),8-tetraen-3-ylsulfamat,
17-Difluoromethylen-15βH,3'H-cycloprop[14,15]-estra-1,3,5(10),8-tetraen-3-ol,
3-Methoxy-15β-methyl-3'H-cycloprop[14,15]-estra-1,3,5(10),8-tetraen-3-ol,
15α-Methyl-3'H-cycloprop[14,15]-estra-1,3,5(10),8-tetraen-3,17α-diol,
17-Difluoromethylen-15βH,3'H-cycloprop[14,15]-estra-1,3,5(10),8-tetraen-3-yl-(tetramethylenimino)sulfonat,
17-Methylen-3'H-cycloprop[8,9]-15βH,3'H-cycloprop[14,15]-estra-1,3,5(10)-trien-3-ol.

Eine vorteilhafte Ausgestaltung der Erfindung ist die erfindungsgemäße Verwendung der Verbindungen zur Herstellung pharmazeutischer Präparate zur Prophylaxe und Therapie der altersabhängigen Verminderung der kognitiven Leistung, der altersbedingten und perimenopausalen Dysphorie, des premenstruellen Syndroms, der Neurose und Neurasthenie, von Angstzustände und -neurosen, von Hitzewallungen nach Estrogen-Deprivation (Menopause, Gonadektomie, Behandlung mit GnRH-Analoga) und der psychogenen Hemmung des Sexualverhaltens.
Es wurde festgestellt, daß dabei das Risiko einer Beeinträchtigung hormonsensitiver Gewebe des Reproduktionssystems (Endometrium, Myometrium, Prostata, Brustdrüse) im Sinne einer unkontrollierter Proliferation ind Karzinogenese weitgehend ausgeschlossen werden kann.

Gegenstand der vorliegenden Erfindung sind auch pharmazeutische Präparate zur oralen und parenteralen, incl. topischen, rektalen, subcutanen, intravenösen, intramuskulären, intraperitonealen, intranasalen, intravaginalen, intrabukkalen oder sublingualen Applikation, die neben üblichen Träger- und Verdünnungsmitteln eine in dem Anspruch 1 aufgezeigte Verbindung als Wirkstoff enthalten.

Als pharmazeutische Formulierungen können zur Anwendung kommen:
- Tabletten oder Dragees von 0,1 bis 2 mg täglich oral,
- Ampullen von 0,1 bis 2 mg täglich als subkutane Injektion,
- Pflaster mit transdermaler Freisetzung von 0,05 bis 2 mg täglich,
- subkutane Implantate mit täglicher Freisetzungskapazität von 0,05 bis 2 mg,
- Gele und Cremen mit transdermaler Freisetzung von 0,05 bis 2 mg täglich,
- bukkal applizierbare Systeme mit einer täglichen Freisetzung von 0,1 bis 1 mg.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Am Beispiel von 15βH,3'H-Cycloprop[14,15]-estra-1,3,5(10),8-tetraen-3,17α-diol (Verbindung der allgemeinen Formel I:

R₁ = R₂ = R₃ = R₄ = R₅ = R₆ = R₇ = H; R₈ = α-OH, β-H; R₉ = CH3;

Z=C,C-Doppelbindung) - nachfolgend in den Figuren als Prototypsubstanz dokumentiert- wird die erfindungsgemäße selektive estrogenähnliche Wirkung experimentell im Vergleich mit 17b-Estradiol und 17a-Estradiol nachgewiesen.

### Beispiel 1

### Einfluß auf das Uterusgewicht nach chronischer subkutaner Applikation in vivo

Geschlechtsreife (drei Monate alt, Gewicht 250±30 g) weibliche Wistar-Ratten (Tierzucht Schönwalde GmbH, Deutschland) wurden unter Ketamin-Narkose ovarektomiert. Nach 14 Tagen wurden die Tiere subkutan mit osmotischen Minipumpen (Alzett, USA) implantiert, die eine Tagesdosis von 0,01, 0,1, 0,3, 1, 3, 30 und 100 µg der zu untersuchenden Substanzen (15βH,3'H-Cycloprop[14,15]-estra-1,3,5(10),8-tetraen-3,17α-diol, 17b-estradiol und 17a-estradiol) über 7 Tage freisetzten; die Kontrolltiere erhielten ein entsprechendes Volumen an Vehikel (Propylenglykol). Am 7. Behandlungstag wurden die Tiere getötet und die Uterus-Feuchtgewichte (bezogen auf 100 g Körpergewicht) ermittelt.

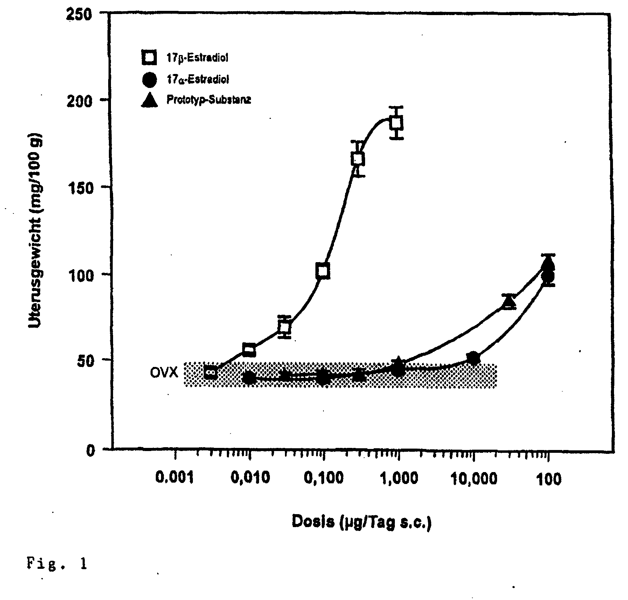

Fig. 1 zeigt die uterotrope Wirkung von verschiedenen Dosen von 17b-Estradiol (Rechteck-Symbole), 17a-Estradiol (Kreis-Symbole) und 15βH,3'H-Cycloprop[14,15]-estra-1,3,5(10),8-tetraen-3,17α-diol, (Dreieck-Symbole) bei ovariektomierten Ratten. Jeder Punkt stellt den Mittelwert ± Standardfehler (x ± SEM) von 7-10 Versuchstieren dar; das schattierte Feld zeigt die Streuungsbreite dieses Parameters in Placebo-behandelten Tieren (OVX).

Es ist ersichtlich, daß mit 17b-Estradiol eine signifikante Vergrößerung des Uterus bereits bei Tagesdosen von 0,03 bis 0,1 µg erzielt wurde. Für einen vergleichbaren uterotropen Effekt benötigte man Tagesdosen von 100 µg 17a-Estradiol bzw. 30 µg der Substanz 15βH,3'H-Cycloprop[14,15]-estra-1,3,5(10),8-tetraen-3,17α-diol.
Dieses Ergebnis zeigt, daß die Wirksamkeit von 15βH,3'H-Cycloprop[14,15]-estra-1,3,5(10),8-tetraen-3,17α-diol im weiblichen Genitaltrakt etwa 1000-fach geringer als diese von 17b-Estradiol und vergleichbar mit derjenigen von 17a-Estradiol ist.

### Beispiel 2

### Aktivierung der Transkription eines a-Estrogenrezeptor-abhängigen Reportergens in vitro

Brustkrebszellen MCF-7/2A, die die Alpha-Isoform des Estrogenrezeptors (ERa) exprimieren, wurden mit dem Reporter-Plasmid EREwtcLUC stabil transfiziert. Der Reporter enthält den Estrogen-Response Element (ERE) von Vitellogenin, einen Thymidinkinase-Promotor und das Luciferase-codierende Gen von *Photinus pyralis*. Die Zellkultur wurde für 7 Tage vor Beginn des Experiments in steroid-freiem Medium kultiviert und anschließend mit 17b-Estradiol, 17a-Estradiol bzw. der Substanz 15βH,3'H-Cycloprop[14,15]-estra-1,3,5(10),8-tetraen-3,17α-diol in vier verschiedenen Konzentra-tionen (10⁻¹¹, 10⁻¹⁰, 10⁻⁹ und 10⁻⁸ M) für 48 Stunden inkubiert. Die Zellen wurden lysiert und die Transkription des Luciferase-Reportergens wurde durch Bestimmung der Luciferase-Aktivität mit einem spezifischen Testansatz (Serva/Promega, Deutschland) ermittelt.

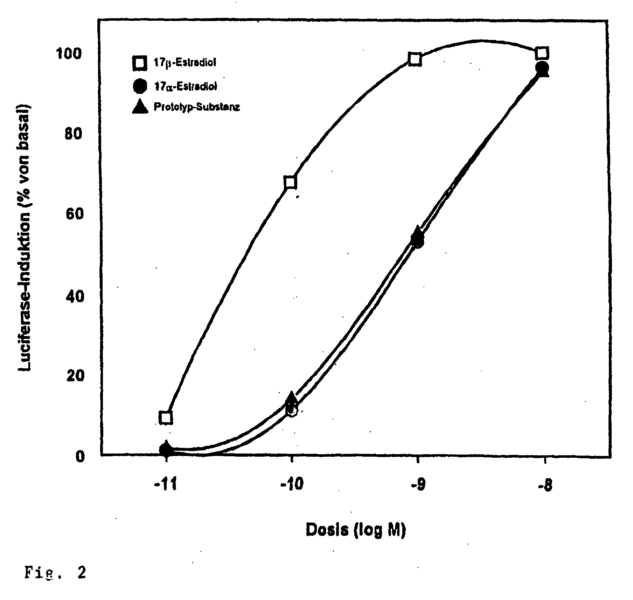

Fig.2 zeigt die Induktion der Transkription eines stabil transfiziertenestrogen-abhängigen Reporter-Gens (Luciferase) in Estrogenrezeptorexprimierenden Brustkrebszellen MCF-7 nach 48-stündiger Behandlung mit verschiedenen Dosen von 17b-Estradiol (Rechteck-Symbole), 17a-Estradiol (Kreis-Symbole) und 15βH,3'H-Cycloprop[14,15]-estra-1,3,5(10),8-tetraen-3,17α-diol (Dreieck-Symbole). Die Figur stellt die Mittelwerte von zwei unabhängigen Versuchen dar.

Es ist ersichtlich, daß die untersuchten Substanzen dosis-abhängig die Transkription des Reporters stimulieren. Die Effizienz von 15βH,3'H-Cycloprop[14,15]-estra-1,3,5(10),8-tetraen-3,17α-diol und 17a-Estradiol ist um eine Größenordnung (d.h. 10-fach) geringer als diese von 17b-Estradiol.
Dieses Ergebnis zeigt, daß die Substanz 15βH,3'H-Cycloprop[14,15]-estra-1,3,5(10),8-tetraen-3,17α-diol eine um das mehrfache schwächere Estrogenwirkung in Brustkrebs-Gewebe hat.

### Beispiel 3

### Stimulation der Transkription des Oxytocin-Gens im Gehirn nach chronischer Behandlung in vivo mit Dosen, die am Uterus unwirksam sind

Geschlechtsreife (drei Monate alt, Gewicht 250±30 g) weibliche Wistar-Ratten (Tierzucht Schönwalde GmbH, Deutschland) wurden unter Ketamin-Narkose ovarektomiert. Nach 14 Tagen wurden die Tiere subkutan mit osmotischen Minipumpen (Alzett, USA) implantiert, die eine Tagesdosis von 0,01, 0,1 und 1 µg der zu untersuchenden Substanzen (15βH,3'H-Cycloprop[14,15]-estra-1,3,5(10),8-tetraen-3,17α-diol, 17b-estradiol und 17a-estradiol) über 7 Tage freisetzten; die Kontrolltiere erhielten ein entsprechendes Volumen an Vehikel (Proplenglykol). Unmittelbar nach der Tötung der Tiere, wurden die Uterus-Feuchtgewichte (bezogen auf 100 g Körpergewicht) ermittelt. Die Messenger-Ribonukleinsäure (mRNA), die die Biosynthese von Oxytozin codiert, wurde durch in-situ-Hybridisierung mit einer spezifischen radioaktiv-markierten Oligodeoxynukleotid-Sonde nach einer etablierten Methode (Fischer D et al, Lactation as a model of naturally reversible hypercorticalism: plasticity in the mechanism governing hypothalamo-pituitary-adrenal activity in the rat, J Clin Invest 96: 1208-1215, 1995), 1995) im hypothalamischen Nucleus paraventricularis (PVN) dargestellt. Behandlungsbedingte Veränderungen in der Transkription des Oxytocin-Gens wurden durch densitometrische Messungen der spezifischen Hybridisierungssignale innerhalb der definierten anatomischen Stukturen quantifiziert.

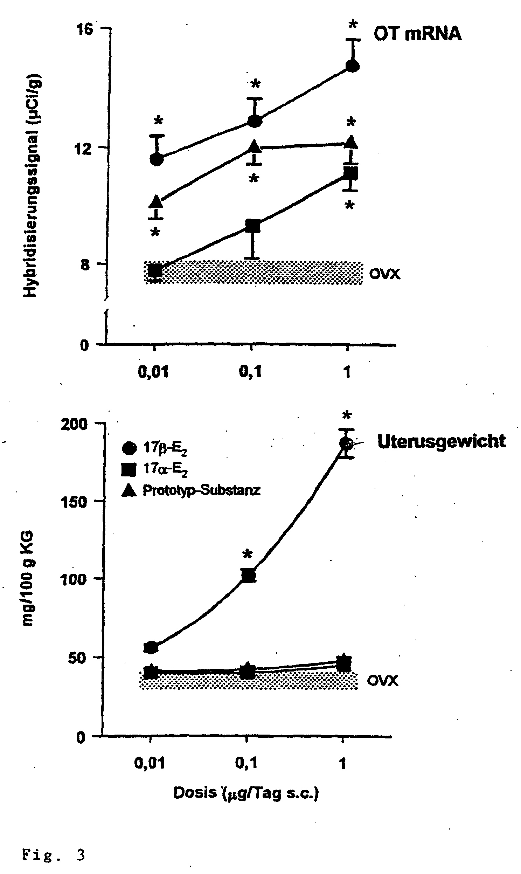

Fig. 3 zeigt die Induktion von Oxytozin-codierenden Transkripten (OT mRNA; obere Graphik) im hypothalamischen paraventrikulären Nukleus ovariektomierter Ratten nach chronischer subkutaner Behandlung mit 17b-Estradiol (Kreis-Symbole), 17a-Estradiol (Rechteck-Symbole) und 15βH,3'H-Cycloprop[14,15]-estra-1,3,5(10),8-tetraen-3,17α-diol (Dreieck-Symbole) in drei verschiedenen Dosierungen. Die untere Graphik zeigt die Effekte der getesteten Substanzen auf das Uterusgewicht. Jeder Punkt stellt x ± SEM von 5-7 Einzelbestimmungen. Das schattierte Feld zeigt die Streuungsbreite des entsprechenden Parameters in Vehikel-behandelten Ratten. Die Sternzeichen bezeichnen signifikante Unterschiede (p<0,05) im Vergleich zu der Kontrollgruppe (OVX).

Die Ergebnisse zeigen, daß 15βH,3'H-Cycloprop[14,15]-estra-1,3,5(10),8-tetraen-3,17α-diol die Transkription des Oxytozin-Gens im PVN dosisabhängig stimuliert, wobei der Stimulationseffekt demjenigen von 17b-Estradiol sehr ähnlich ist. Allerdings sind die neurotropen Transkriptionseffekte von 15βH,3'H-Cycloprop[14,15]-estra-1,3,5(10),8-tetraen-3,17α-diol, unterschiedlich als bei 17b-Estradiol, mit keiner Uterus-Vergrößerung assoziiert. In den verwendeten Dosierungen hatte 17a-Estradiol keinen Einfluß auf die Konzentrationen von OxytozinmRNA im hypothalamischen PVN.

Diese Ergebnisse dokumentieren eine selektive estrogen-ähnliche Wirkung von 15βH,3'H-Cycloprop[14,15]-estra-1,3,5(10),8-tetraen-3,17α-diol im Gehirn der weiblichen Ratte.

### Beispiel 4

### Stimulation der Transkription des antiapoptotischen Gens bcl-2 im Hippokampus nach chronischer Behandlung in vivo mit Dosen, die keine uterotrope Wirkung zeigen

Das Untersuchungsmaterial stammte von Tieren, die im unter Beispiel 3 beschriebenen Experiment behandelt wurden. Das Gen bcl-2 codiert die Synthese eines Proteins, das in der Kaskade der Zellproliferation involviert ist und dem programmierten Zelltod (Apoptose) entgegenwirkt (Merry DE, Korsmeyer SJ, Bcl-2 gene family in the nervous system, Ann Rev Neurosci 20: 245-267, 1997). Die Transkription dieses Gens wird durch Estrogene stimuliert (Kandouz M et al., Antagonism between estradiol and progestin on Bcl-2 expression in breast cancer cells, Int J Cancer 68: 120-125, 1996).
Gyrus dentatus ist ein Bestandteil der hippokampalen Formation, in dem die Neurogenese bei der Ratte auch im Erwachsenenalter persistiert (Gould E et al, Proliferation of granule cell precursors in the dentate gyrus of adult monkeys is diminished by stress, Proc Natl Acad Sci USA 96: 3168-317, 1998) und bcl-2 exprimiert ist. Bcl-2-Transkripte wurden in Hirnschnitten durch in-situ-Hybridisierung mit einer spezifischen Oligonukleotid-Sonde (Clark RSB et al., Apoptosis-suppressor gene bcl-2 expression after traumatic brain injury in rats, J Neurosci 17: 9172-9182, 1997) dargestellt, und nach der im Beispiel 3 beschriebenen Methode densitometrisch quantifiziert.

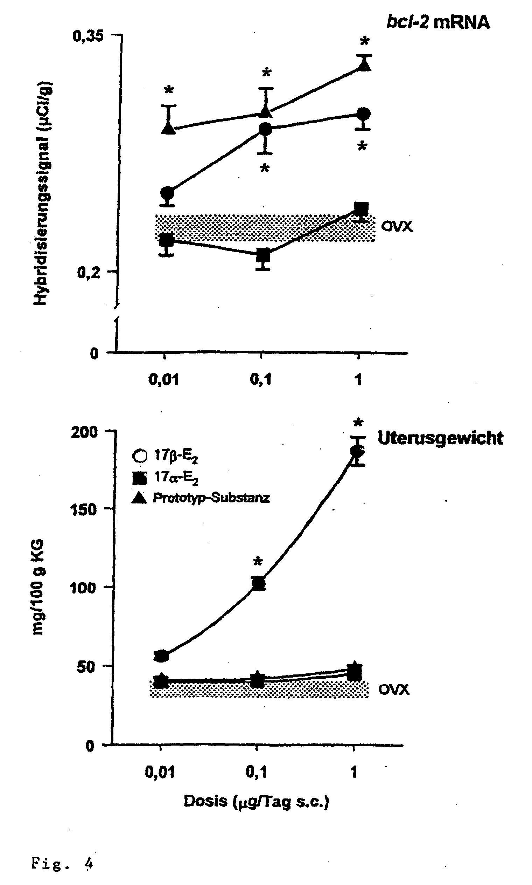

Fig. 4 zeigt den Einfluß von drei verschiedenen Dosen von 17b-Estradiol (Kreis-Symbole), 17a-Estradiol (Rechteck-Symbole) und 15βH,3'H-Cycloprop[14,15]-estra-1,3,5(10),8-tetraen-3,17α-diol (Dreieck-Symbole) auf die Expression von bcl-2 im Gyrus dentatus des Hippokampus ovariektomierter Ratten; Zeichen und Abkürzungen wie in Fig. 3.

Die Behandlung mit der Substanz 15βH,3'H-Cycloprop[14,15]-estra-1,3,5(10),8-tetraen-3,17α-diol resultierte in einer dosisabhängigen Stimulation der Expression von bcl-2 im Gyrus dentatus. Der Effekt war mit demjenigen, der durch gleiche Dosen 17b-Estradiol ausgelöst wurde, identisch. Die Substanz 17a-Estradiol hatte in den verwendeten Dosierungen keinen Effekt auf die Transkription von bcl-2 - ersichtlich aus Figur 4.
Diese Ergebnisse zeigen, daß in der verwendeten Dosierung, 15βH,3'H-Cycloprop[14,15]-estra-1,3,5(10),8-tetraen-3,17α-diol die Transkription des antiapoptotischen Gens bcl-2 im ZNS nach einem estrogen-ähnlichen Modus beeinflußt, ohne daß eine Wirkung am Uterus auftritt.

### Beispiel 5

### Dissoziierte Induktion von Oxytozin-Rezeptoren im Gehirn und im Myometrium

Bindungsstellen mit identischen biochemischen Charakteristika für das Peptidhormon Oxytozin sind im Myometrium und im ZNS vorhanden. In beiden Organen verursacht akute oder chronische Estrogen-Behandlung einen Anstieg der Anzahl (Dichte) von Oxytozin-Rezeptoren. Die Hirnstrukturen, in denen dieser Parameter besonders empfindlich auf Estrogene reagiert, sind Nucleus interstitialis striae terminalis, Nucleus ventromedialis und der amygdaloide Nuklearkomplex. Estrogen-bedingte Induktion von Oxytozin-Rezeptoren in diesen Strukturen befindet sich in einer kausalen Relation zur Ausprägung von einer Reihe von prosozialen Verhaltensmustern, darunter auch Sexualverhalten (Insel TR, Oxytocin - a neuropeptide for affiliation: evidence from behavioral, receptor autoradiographic, and comparative studies, Psychoneuroendocrinology 17: 3-35, 1992).

Zu Bestimmungen der Dichte von Oxytozin-Rezeptoren in definierten anatomischen Strukturen ist die autoradiographische Darstellung durch Bindung des radioaktiv markierten Oxytozin-Rezeptorantagonisten d(CH₂)₅-Tyr(Me)², Thr⁴, Orn⁸-[¹²⁵I]Tyr⁹-Vasotocin (¹²⁵I-OVTA) die Methode der Wahl (Kremarik P et al., Histoautoradiographic detection of oxytocin- and vasopressin-binding sites in the telencephalon of the rat, J Comp Neurol 333: 343-359, 1993).
Gefrierschnitte vom Gehirn und Uterus von ovariektomierten Ratten, die 7 Tage lang eine tägliche subkutane Dosis von 1 µg 15βH,3'H-Cycloprop[14,15]-estra-1,3,5(10),8-tetraen-3,17α-diol, 17b-Estradiol bzw. 17a-Estradiol erhielten (vgl. Beispiel 3), wurden mit ¹²⁵I-OVTA (NEN DuPont, Deutschland) in einer Konzentration von 50 pM inkubiert. Anschließend wurden Filmautoradiogramme erstellt, die zur densitometrischen Bestimmung der Oxytozin-Bindungsstellen nach einem etablierten Verfahren verwendet wurden (Patchev VK et al., Oxytocin binding sites in rat limbic and hypothalamic structures: site-specific modulation by adrenal and gonadal steroids. Neuroscience 57: 537-543, 1993).

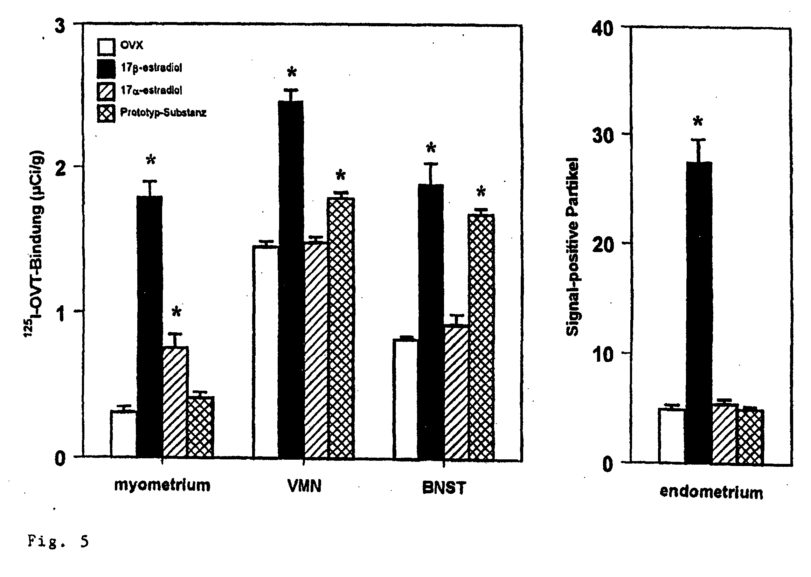

Fig. 5 zeigt die spezifische Bindung eines ¹²⁵I-markierten Liganden des Oxytozin-Rezeptors (¹²⁵I-OVT) im Myometrium und in zwei estrogen-sensitiven Hirnstrukturen, dem hypothalamischen ventromedialen Nukleus (VMN) und dem Nucleus interstitialis striae terminalis (BNST), nach einer 7-tägigen Behandlung mit 17b-Estradiol (schwarze Säulen), 17a-Estradiol (schraffierte Säulen) und 15βH,3'H-Cycloprop[14,15]-estra-1,3,5(10),8-tetraen-3,17α-diol (graue Säulen) in einer Tagesdosis von 1 µg. Die Sternzeichen zeigen signifikante Unterschiede (p<0,05) im Vergleich zu Placebo-behandelten ovariektomierten Ratten (OVX). Die rechte Graphik stellt die Effekte der getesteten Substanzen auf die Proliferation des Endometriums dar. Jede Säule repräsentiert x ± SEM von 4-5 Einzelbestimmungen.

Die Ergebnisse dieser Untersuchung sind ebenfalls auf Fig. 5 dargestellt. Die Behandlung mit 17b-Estradiol und 15βH,3'H-Cycloprop[14,15]-estra-1,3,5(10),8-tetraen-3,17α-diol resultierte in einem signifikanten Anstieg der Dichte von Oxytozin-Rezeptoren in allen untersuchten Hirnstrukturen, wobei 15βH,3'H-Cycloprop[14,15]-estra-1,3,5(10),8-tetraen-3,17α-diol im VMN einen schwächeren Effekt als 17b-Estradiol zeigte. Die Substanz 17a-Estradiol war bei der verwendeten Dosierung in keiner Hirnstruktur wirksam. Im Myometrium verursachte 17b-Estradiol eine starke Induktion von Oxytozin-Bindungsstellen, während 17a-Estradiol und die Substanz 15βH,3'H-Cycloprop[14,15]-estra-1,3,5(10),8-tetraen-3,17α-diol eine signifikant geringere Wirkung zeigten. Die computer-gestützte Messung der Stärke des Endometriums in den Uteruspräparaten zeigte, daß die Tagesdosis von 1 µg 17b-Estradiol eine signifikante endometriale Proliferation verursacht, während 15βH,3'H-Cycloprop[14,15]-estra-1,3,5(10),8-tetraen-3,17α-diol und 17a-Estradiol in einer äquivalenten Dosierung die Stärke des Endometriums nicht beeinflussen.
Zusammenfassend zeigen die unter Beispiel 5 dargestellten Resultate, daß die Substanz 15βH,3'H-Cycloprop[14,15]-estra-1,3,5(10),8-tetraen-3,17α-diol einen biochemischen Parameter - den Oxytozin-Rezeptor - der sowohl für das reproduktive System (Myometrium) als auch für das ZNS charakteristisch ist, vorwiegend im Zentralnervensystem beeinflusst und sich durch diese selektive neurotrope Wirkung von den natürlichen Estrogenen 17b-Estradiol und 17a-Estradiol qualitativ unterscheidet.

### Beispiel 6

### Beeinflussung der kognitiven Funktionen nach chronischer Behandlung

Es ist bekannt, daß eine Verminderung der Estrogenkonzentrationen mit einer Erniedrigung der Lern- und Gedächtnisteistung assoziiert ist (Kopera H, Estrogens and psychic functions. Aging and estrogens, Front Hormone Res. 2: 118-133, 1973).

Eine Korrelation zwischen Serum-Estrogenspiegel und kognitiver Leistung wurde auch im Tierversuchsmodell nachgewiesen (Kondo Y, Suzuki K, Sakuma Y, Estrogen aleviates cognitive dysfunction following transient brain ischemia in ovariectomized gerbils, Neurosci Lett 238: 45-48, 1997).
Zur Vergleichsuntersuchung des Einflusses der Substanzen 15βH,3'H-Cycloprop[14,15]-estra-1,3,5(10),8-tetraen-3,17α-diol, 17b-Estradiol und 17a-Estradiol auf die kognitive Leistung wurde das folgende Experiment durchgeführt:
Geschlechtsreife weibliche Wistar-Ratten (Gewicht 240±20 g) wurden unter Nembutal-Narkose ovarektomiert. Eine Woche nach der Operation wurde mit täglicher subkutaner Verabreichung der Substanzen in den folgenden Tagesdosen begonnen: 17b-Estradiol, 1 µg; 17a-Estradiol, 100 µg; 15βH,3'H-Cycloprop[14,15]-estra-1,3,5(10),8-tetraen-3,17α-diol, 30 µg. Die gesamte Behandlungsdauer war 14 Tage. Am 5. und 6. Behandlungstag wurden Trainingssitzungen zum Erlernen eines konditionierten Escape-Verhaltens nach einer etablierten Methode (Diaz-Veliz G et al., Influence of the estrous cycle, ovariectomy and estradiol replacement upon the acquisition of conditioned avoidance responses in rats, Physiol Behav 46: 397-401, 1989) durchgeführt. Jedes Tier wurde in einer Sitzung 50 Mal der Kombination aus einem unbedingten (elektrische Reizung) und zwei konditionierenden Stimuli (Licht- und Schall-Signal) exponiert. Am 7. Behandlungstag wurde die Retention des erlernten Verhaltensmusters getestet. Nach einer 6-tägigen Unterbrechung der Lernsitzungen, wurde am 14. Behandlungstag die Extinktion des erlernten konditionierten Verhaltens ermittelt. Die Anzahl der korrekten Verhaltensreaktionen (Escape in das "sichere" Abteil des Apparats innerhalb von drei Sekunden nach Präsentation der konditionierenden Signale) aus 50 aufeinanderfolgenden Expositionen wurde als Kriterium für die Bewertung der Retention bzw. Extinktion des erlernten Verhaltens verwendet.

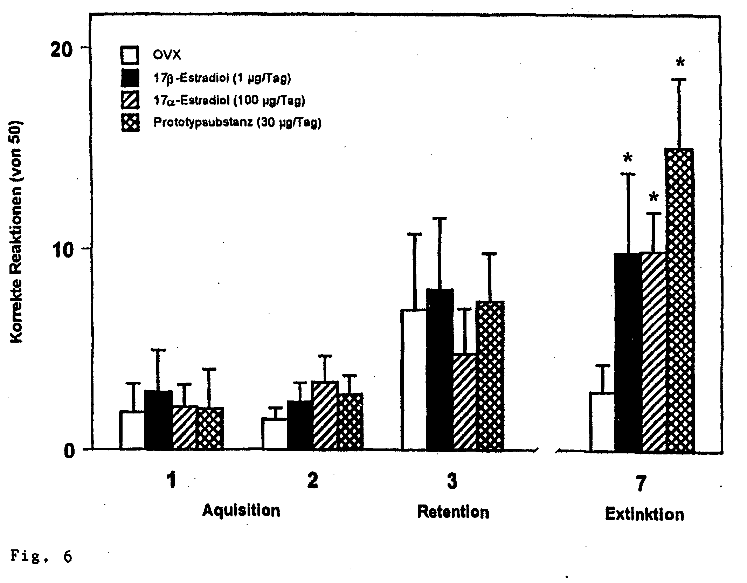

Fig. 6 zeigt den Einfluß von 17b-Estradiol (schwarze Säulen), 17a-Estradiol (schraffierte Säulen) und 15βH,3'H-Cycloprop[14,15]-estra-1,3,5(10),8-tetraen-3,17α-diol (graue Säulen) auf die Aquisition und Retention eines neuen Verhaltensmusters bei ovariektomierten Ratten (offene Säulen; OVX). Die Sternzeichen zeigen signifikante Unterschiede im Vergleich zu den Placebo-behandelten Tieren (OVX) am entsprechenden Testtag. Die folgenden Uterusgewichte (x ± SEM; n = 8-10 pro Behandlungsgruppe; Angaben in mg/100g KG) wurden nach 14-tägiger Behandlung ermittelt: OVX, 53 ± 2; 17b-Estradiol, 187 ± 9; 17a-Estradiol, 100 ± 5; 15βH,3'H-Cycloprop[14,15]-estra-1,3,5(10),8-tetraen-3,17α-diol, 108 ± 4.

Es ist ersichtlich, daß die Substanz 15βH,3'H-Cycloprop[14,15]-estra-1,3,5(10),8-tetraen-3,17α-diol in der verwendeten Dosierung einen estrogen-ähnlichen stimulierenden Effekt auf die Retention des erlernten Verhaltensmusters hat, wobei die uterotrope Wirkung signifikant geringer ist, als diese von 17b-Estradiol in einer täglichen Dosis von 1 µg. Dieses Ergebnis weist nach, daß 15βH,3'H-Cycloprop[14,15]-estra-1,3,5(10),8-tetraen-3,17α-diol die kognitive Leistung wie ein Estrogen beeinflußt, während in reproduktiven Organen eine geringfügige proliferative Wirkung zeigt.

### Beispiel 7

### Biotransformation von17a-Hydroxy-14,15a-methylen-estra-,3,5 (10),8-tetraen-3-ol und 17a-Estradiol zu 17b-Estradiol

Ovariektomierte Ratten erhielten tägliche subkutane Dosen von 100 µg 17a-Estradiol, 30 µg 15βH,3'H-Cycloprop[14,15]-estra-1,3,5(10),8-tetraen-3,17α-diol bzw. 1 µg 17b-Estradiol über 7 Tage (vgl. Beispiel 6). Am letzen Behandlungstag wurden die Serumkonzentrationen von 17b-Estradiol im den drei Versuchsgruppen ermittelt und mit denenigen bei vehikel-behandelten Kontrolltieren verglichen.

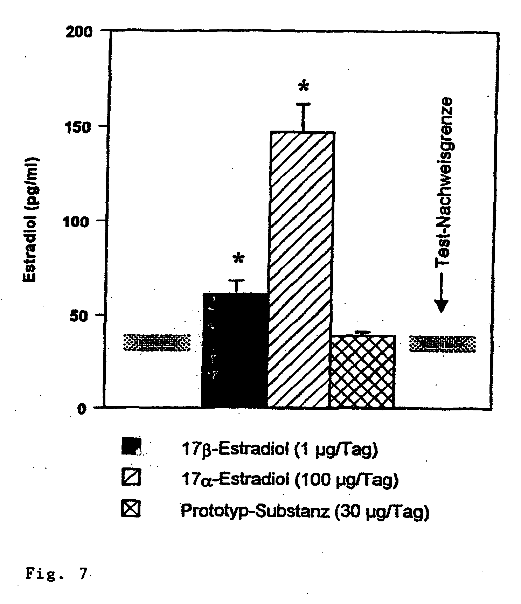

Fig. 7 zeigt Serumwerte von 17b-Estradiol nach einer 7-tägiger subkutaner Behandlung ovariektomierter Ratten mit 17b-Estradiol (schwarze Säule), 17a-Estradiol (schraffierte Säule) und 15βH,3'H-Cycloprop[14,15]-estra-1,3,5(10),8-tetraen-3,17α-diol (graue Säule) in den angegebenen Dosierungen. Sternzeichen bezeichnen signifikante Differenzen im Vergleich zu den Werten, die bei Placebo-behandelten Tieren gemessen wurden; die Letzteren waren unterhalb der Nachweisgrenze der Methode; jede Behandlungsgruppe bestand aus 7 Tieren.

Es ist ersichtlich, daß nach der Applikation von 17b-Estradiol und 17a-Estradiol in den erwähnten Dosierungen meßbare Konzentrationen von 17b-Estradiol im Serum registriert werden. Chronische subkutane Behandlung mit 15βH,3'H-Cycloprop[14,15]-estra-1,3,5(10),8-tetraen-3,17α-diol verursacht keinen Anstieg der endogenen 17b-Estradiolspiegel. Dieses Ergebnis weist darauf, daß die beobachteten pharmakologischen Effekte nach der Verabreichung von 15βH,3'H-Cycloprop[14,15]-estra-1,3,5(10),8-tetraen-3,17α-diol nicht auf eine Biotransformation der Substanz zu 17b-Estradiol zurückzuführen sind.

## Patentansprüche

1. Verwendung von Steroiden der allgemeinen Formel I 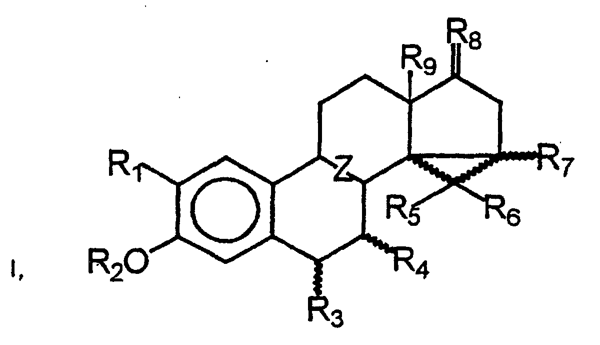 wobei
R₁ ein Wasserstoffatom, eine Hydroxylgruppe oder eine Alkyloxygruppe von 1 bis 5 C-Atomen darstellt,
R₂ ein Wasserstoffatom, eine Alkylgruppe von 1 bis 5 C-Atomen, eine Acylgruppe von 1 bis 5 C-Atomen, eine Gruppierung der allgemeinen Formel SO₂NR₁₀R₁₁,
wobei R₁₀ und R₁₁ unabhängig voneinander jeweils ein Wasserstoffatom, eine Alkylgruppe von 1 bis 5 C-Atomen oder zusammen mit dem Stickstoff eine Pyrrolidino-, Piperidino- oder Morpholinogruppe bedeuten,
R₃ ein Wasserstoffatom oder eine Hydroxylgruppe darstellt,
R₄ ein Wasserstoffatom, eine Hydroxylgruppe oder eine Alkylgruppe bis 5 C-Atomen bedeutet,
R₅ und R₆ unabhängig voneinander jeweils ein Wasserstoffatom oder ein Halogenatom bedeuten,
R₇ für ein Wasserstoffatom oder eine Methylgruppe steht,
R₈ ein Wasserstoffatom und eine Hydroxylgruppe, eine Oxogruppe
oder eine Gruppierung der allgemeinen Formel CR₁₂R₁₃ bedeutet,
in der R₁₂ und R₁₃ unabhängig
voneinander jeweils ein Wasserstoffatom oder ein Halogenatom darstellen,
R₉ eine Methyl- oder Ethylgruppe bedeutet,
Z für eine C,C-Doppelbindung oder einen substituierten oder unsubstituierten Cyclopropanring steht
und die Gruppierung >CR₅R₆ entweder α-oder β-ständig angeordnet ist, wobei R₇ β-ständig ist, wenn >CR₅R₆ α-ständig ist und umgekehrt,
zur Herstellung pharmazeutischer Präparate zur gezielten Substitution des Estrogenmangels im Zentralnervensystem (ZNS) ohne Beeinflussung anderer Organe oder Systeme.

2. Verwendung von Steroiden nach Anspruch1,
wobei diese Steroide
15βH,3'H-Cycloprop[14,15]-estra-1,3,5(10),8-tetraen-3,17α-diol,
15βH,3'H-Cycloprop[14,15]-18a-homo-estra-1,3,5(10),8-tetraen-3,17α-ol,
17α-Hydroxy-15βH,3'H-cycloprop[14,15]-estra-1,3,5(10),8-tetraen -3-yl-pentanoat,
17-Methylen-15βH,3'H-cycloprop[14,15]-estra-1,3,5(10),8-tetraen-3-ol,
15βH,3'H-3',3'-difluoro-cycloprop[14,15]-estra-1,3,5(10),8-tetraen-3,17α-diol,
17-Methylen-15βH,3'H-cycloprop[14,15]-estra-1,3,5(10),8-tetraen-3-yl-sulfamat,
17-Difluoromethylen-15βH,3'H-cycloprop[14,15]-estra-1,3,5(10),8-tetraen-3-ol,
3-Methoxy-15β-methyl-3'H-cycloprop[14,15]-estra-1,3,5(10),8-tetraen-3-ol,
15α-Methyl-3'H-cycloprop[14,15]-estra-1,3,5(10),8-tetraen-3,17α-diol,
17-Difluoromethylen-15βH,3'H-cycloprop[14,15]-estra-1,3,5(10),8-tetraen-3-yl-(tetramethylenimino)sulfonat,
17-Methylen-3'H-cycloprop[8,9]-15βH,3'H-cycloprop[14,15]-estra-1,3,5(10)-trien-3-ol.

3. Verwendung von Steroiden nach Anspruch 1 und 2
zur Herstellung pharmazeutischer Präparate zur Prophylaxe und Therapie
der altersabhängigen Verminderung der kognitiven Leistung,
der altersbedingten und perimenopausalen Dysphorie,
des premenstruellen Syndrom,
von Neurose und Neurasthenie,
von Angstzuständen und -neurosen,
von Hitzewallungen nach Estrogen-Deprivation (Menopause, Gonadektomie, Behandlung mit GnRH-Analoga),
der psychogenen Hemmung des Sexualverhaltens.

## Claims

1. Use of steroids of the general formula I 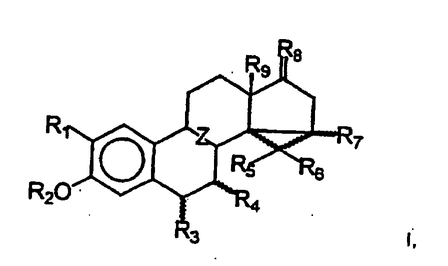 wherein
R₁ represents a hydrogen atom, a hydroxyl group or an alkoxy group having 1 to 5 carbon atoms,
R₂ represents a hydrogen atom, an alkyl group having 1 to 5 carbon atoms, an acyl group having 1 to 5 carbon atoms or a moiety of the general formula SO₂NR₁₀R₁₁,
wherein R₁₀ and R₁₁ represent, independently of each other, a hydrogen atom, an alkyl group having 1 to 5 carbon atoms, or together with nitrogen, a pyrrolidino, piperidino or morpholino group,
R₃ represents a hydrogen atom or a hydroxyl group,
R₄ represents a hydrogen atom, a hydroxyl group or an alkyl group to 5 carbon atoms,
R₅ and R₆ represent, independently of each other, a hydrogen atom or a halogen atom,
R₇ represents a hydrogen atom or a methyl group,
R₈ represents a hydrogen atom and a hydroxyl group, and oxo group or a moiety of the general formula CR₁₂R₁₃,
in which R₁₂ and R₁₃ represent, independently of each other, a hydrogen atom or a halogen atom,
R₉ represents a methyl or ethyl group,
Z represents a C-C double bond or a substituted or unsubstituted cyclopropane ring
and the moiety >CR₅R₆ is either α- or β-disposed, with the proviso that R₇ is β-disposed when >CR₅R₆ is α-disposed, and vice versa,
for manufacturing pharmaceutical products for targeted replacement for oestrogen deficiency in the central nervous system (CNS) without affecting other organs or systems.

2. Use of steroids according to Claim 1, wherein these steroids
15βH,3'H-cycloprop[14,15]-estra-1,3,5(10),8-tetraene-3,17 -diol,
15βH,3'H-cycloprop[14,15]-18 -homoestra-1,3,5(10),8-tetraene-3,17 -diol,
17 -hydroxy-15βH,3'H-cycloprop[14,15]-estra-1,3,5(10),8-tetraen-3-yl pentanoate,
17-methylene-15βH,3'H-cycloprop[14,15]-estra-1,3,5(10),8-tetraen-3-ol,
15βH,3'H-3',3'-difluorocycloprop[14,15]-estra-1,3,5(10),8-tetraene-3,17 -diol,
17-methylene-15βH,3'H-cycloprop[14,15]-estra-1,3,5(10),8-tetraen-3-yl sulphamate,
17-difluoromethylene-15βH,3'H-cycloprop[14,15]-estra-1,3,5(10),8-tetraen-3-ol,
3-methoxy-15β-methyl-3'H-cycloprop[14,15]-estra-1,3,5(10),8-tetraen-3-ol,
15 -methyl-3'H-cycloprop[14,15]-estra-1,3,5(10),8-tetraene-3,17 -diol,
17-difluoromethylene-15βH,3'H-cycloprop[14,15]-estra-1,3,5(10),8-tetraen-3-yl (tetramethyleneimino)sulphonate and
17-methylene-3'H-cycloprop[8,9]-15βH,3'H-cycloprop[14,15]-estra-1,3,5(10)-trien-3-ol.

3. Use of steroids according to Claim 1 and 2 for manufacturing pharmaceutical products for prophylaxis and therapy
of age-dependent reduction in cognitive performance
of age-based and perimenopausal dysphoria,
of premenstrual syndrome,
of neurosis and neurasthenia,
of anxiety states and neuroses,
of hot flushes after oestrogen deprivation (menopause, gonadectomy, treatment with GnRH analogues),
of psychogenic inhibition of sexual behaviour.

## Revendications

1. Utilisation de stéroïdes de formule générale I 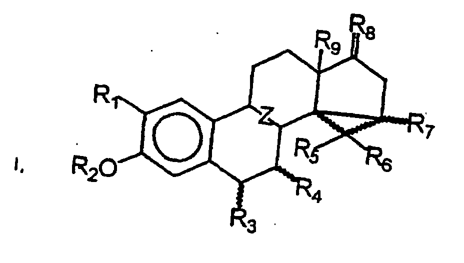 dans laquelle
R₁ représente un atome d'hydrogène, un groupe hydroxy ou un groupe alcoxy de 1 à 5 atomes de carbone,
R₂ représente un atome d'hydrogène, un groupe alkyle de 1 à 5 atomes de carbone, un groupe acyle de 1 à 5 atomes de carbone,
un groupement de formule générale SO₂NR₁₀R₁₁,
R₁₀ et R₁₁ représentant chacun, indépendamment l'un de l'autre, un atome d'hydrogène, un groupe alkyle de 1 à 5 atomes de carbone, ou formant ensemble avec l'atome d'azote un groupe pyrrolidino, pipéridino ou morpholino,
R₃ représente un atome d'hydrogène ou un groupe hydroxy,
R₄ représente un atome d'hydrogène, un groupe hydroxy ou un groupe alkyle ayant jusqu'à 5 atomes de carbone,
R₅ et R₆ représentent chacun, indépendamment l'un de l'autre, un atome d'hydrogène ou un atome d'halogène,
R₇ représente un atome d'hydrogène ou un groupe méthyle,
R₈ représente un atome d'hydrogène et un groupe hydroxy, un groupe oxo
ou un groupement de formule générale CR₁₂R₁₃,
dans lequel R₁₂ et R₁₃ représentent chacun, indépendamment l'un de l'autre, un atome d'hydrogène ou un atome d'halogène,
R₉ représente un groupe méthyle ou éthyle,
Z représente une double liaison C-C ou un cycle cyclopropane substitué ou non substitué
et le groupement >CR₅R₆ est disposé en position soit α, soit β, R₇ étant en position β lorsque >CR₅R₆ est en position α et inversement,
pour la fabrication de préparations pharmaceutiques destinées à pallier de façon ciblée à la carence en oestrogènes dans le système nerveux central (CNS) sans influer sur d'autres organes et systèmes.

2. Utilisation de stéroïdes selon la revendication 1, ces stéroïdes étant
le 15βH,3'H-cycloprop[14,15]-estra-1,3,5(10),8-tétraène-3,17α-diol,
le 15βH,3'H-cycloprop[14,15]-18a-homo-estra-1,3,5(10),8-tétraén-3,17α-ol,
le pentanoate de 17α-hydroxy-15βH,3'H-cycloprop[14,15]-estra-1,3,5(10),8-tétraén-3-yle,
le 17-méthylène-15βH,3'H-cycloprop[14,15]-estra-1,3,5(10),8-tétraén-3-ol,
le 15βH,3',3'-difluoro-cycloprop[14,15]-estra-1,3,5(10),8-tétraène-3,17α-diol,
le sulfamate de 17-méthylène-15βH,3'H-cycloprop[14,15]-estra-1,3,5(10),8-tétraén-3-yle,
le 17-difluorométhylène-15βH,3'H-cycloprop[14,15]-estra-1,3,5(10),8-tétraén-3-ol,
le 3-méthoxy-15β-méthyl-3'H-cycloprop[14,15]-estra-1,3,5(10),8-tétraén-3-ol,
le 15α-méthyl-3'H-cycloprop[14,15]-estra-1,3,5(10),8-tétraène-3,17α-diol,
le (tétraméthylène-imino)sulfonate de 17-difluorométhylène-15βH,3'H-cycloprop[14,15]-estra-1,3,5(10),8-tétraén-3-yle,
le 17-méthylène-3'H-cycloprop[8,9]-15βH,3'H-cycloprop[14,15]-estra-1,3,5(10)-trién-3-ol.

3. Utilisation de stéroïdes selon les revendications 1 et 2, pour la fabrication de préparations pharmaceutiques destinées à la prophylaxie et à la thérapie
de la diminution, dépendant de l'âge, de la puissance cognitive,
de la dysphorie due à l'âge et périménopausale,
du syndrome prémenstruel,
de la névrose et de la neurasthénie,
d'états et de névroses d'angoisse,
des bouffées de chaleur après privation d'oestrogènes (ménopause, gonadectomie, traitement par des analogues de GnRH),
de l'inhibition psychogène du comportement sexuel.
